# EUROPEAN PATENT SPECIFICATION

(11) **EP 2 598 134 B1**
(45) Date of publication and mention of the grant of the patent: **16.11.2016**
(21) Application number: 11745856.2
(22) Date of filing: 26.07.2011
(51) Int. Cl.: A61K 31/198, A61K 31/5415, A61P 43/00, A61P 31/04, A61P 11/00, A61P 13/00, A61P 19/00, A61P 35/00, A61P 17/00

(54) **COMPOSITION AND METHOD FOR PHOTODYNAMIC DISINFECTION**
ZUSAMMENSETZUNG UND VERFAHREN FÜR FOTODYNAMISCHE DESINFEKTION
COMPOSITION ET PROCÉDÉ DE DÉSINFECTION PHOTODYNAMIQUE

(30) Priority: 30.07.2010 US 369166 P
(43) Date of publication of application: 05.06.2013
(73) Proprietor: Advanced Photodynamic Technologies, Inc., Bothell, WA 98011 (US)
(72) Inventor: BIEL, Merrill, Mendota Heights, MN 55118 (US)
(74) Representative: Geary, Stephen
(86) International application number: PCT/US2011/045367
(87) International publication number: WO 2012/015820

(56) References cited:
- WO-A1-2006/117399
- WO-A2-01/62289
- US-A1- 2009 093 470
- Z MALIK ET AL: "Photodynamic inactivation of Gram-negative bacteria: Problems and possible solutions", JOURNAL OF PHOTOCHEMISTRY AND PHOTOBIOLOGY B: BIOLOGY, vol. 14, no. 3, 15 July 1992 (1992-07-15), pages 262-266, XP55011273, ISSN: 1011-1344, DOI: 10.1016/1011-1344(92)85104-3
- BERTOLINI G ET AL: "Photosensitizing activity of water- and lipid-soluble phthalocyanines on Escherichia coli", FEMS MICROBIOLOGY LETTERS, BLACKWELL PUBLISHING, AMSTERDAM, NL, vol. 71, no. 1-2, 1 September 1990 (1990-09-01), pages 149-156, XP023918380, ISSN: 0378-1097, DOI: 10.1111/J.1574-6968.1990.TB03814.X [retrieved on 1990-09-01]
- DAI T ET AL: "Photodynamic therapy for localized infections-State of the art", PHOTODIAGNOSIS AND PHOTODYNAMIC THERAPY, ELSEVIER, vol. 6, no. 3-4, 1 September 2009 (2009-09-01), pages 170-188, XP026771086, ISSN: 1572-1000, DOI: 10.1016/J.PDPDT.2009.10.008 [retrieved on 2009-11-18]
- O'RIORDAN K ET AL: "The potential for photodynamic therapy in the treatment of localized infections", PHOTODIAGNOSIS AND PHOTODYNAMIC THERAPY, ELSEVIER, vol. 2, no. 4, 1 December 2005 (2005-12-01), pages 247-262, XP027629012, ISSN: 1572-1000 [retrieved on 2005-12-01]
- LAMBRECHTS S A G ET AL: "Effect of albumin on the photodynamic inactivation of microorganisms by a cationic porphyrin", JOURNAL OF PHOTOCHEMISTRY AND PHOTOBIOLOGY B: BIOLOGY, ELSEVIER SCIENCE S.A., BASEL, CH, vol. 79, no. 1, 4 April 2005 (2005-04-04), pages 51-57, XP025302096, ISSN: 1011-1344, DOI: 10.1016/J.JPHOTOBIOL.2004.11.020 [retrieved on 2005-04-04]
- BERTOLONI G ET AL: "Hematoporphyrin-sensitized photoinactivation of Streptococcus faecalis.", PHOTOCHEMISTRY AND PHOTOBIOLOGY JUN 1984 LNKD- PUBMED:6431458, vol. 39, no. 6, June 1984 (1984-06), pages 811-816, XP002663121, ISSN: 0031-8655

## Description

### FIELD OF THE INVENTION

The present invention relates to a photosensitizer composition having significantly enhanced antimicrobial efficacy for photodynamic disinfection. More particularly, the present invention relates to a photosensitizer composition comprising a photosensitizer and a very low concentration of ethylenediaminetetraacetic acid to enhance the photodynamic disinfection performance of the photosensitizer.

### BACKGROUND OF THE INVENTION

Chronic recurrent sinusitis ("CRS") is an inflammatory disease of the facial sinuses and nasal passages that is defined as lasting longer than 12 weeks or occurring more than 4 times per year with symptoms usually lasting more than 20 days. See Marple BF, Stankiewicz JA, Baroody FM, et al. "Diagnosis and Management of Chronic Rhinosinusitis in Adults." Postgraduate Medicine. 121(6):121-39. The National Institute for Health Statistics estimates that CRS is one of the most common chronic conditions in the United States affecting an estimated 37 million Americans. See National Center for Health Statistics, NCHS. "Chronic sinusitis." In: Summary Health Statistics for US Adults, 2002*.* Hyattsville, MD: Centers for Disease Control, US Department of Health and Human Services, 2002. It is also estimated that CRS results in 18-22 million office visits per year and over 500,000 emergency visits per year resulting in an estimated 73 million restricted activity days with an aggregated cost of six billion dollars annually. See Murphy MP, Fishman P, Short SO, et al. "Health care utilization and cost amount adults with chronic rhinosinusitis enrolled in a health maintenance organization." Otolaryngol Head Neck Surg. 2002; 127(5):367-76; Gliklich RE, Metson R. "The health impact of chronic sinusitis in patients seeking otolaryngologic care." Otolaryngol Head Neck Surg. 1995; 113:104-9.

CRS can present as a headache, facial pain, dental pain, breathing difficulty, purulent nasal drainage, post-nasal drip, hyposmia and/or purulence on nasal examination. See "Diagnosis and Management of Chronic Rhinosinusitis in Adults" cited above. The potential etiologies of CRS include bacteria, viruses, allergies, fungi, superantigens and microbial biofilms. Importantly, CRS is also considered to be a significant factor that can exacerbate asthma, chronic lung diseases, eczema, otitis media and chronic fatigue. See "The health impact of chronic sinusitis in patients seeking otolaryngologic care" cited above; Hamilos DL. "Chronic sinusitis." J Allergy Clin. Immunol. 2000; 106:213-27; Somerville LL. "Hidden factors in asthma." Allergy Asthma Proc. 2001; 22:341-5; Chester AC. "Health impact of chronic sinusitis." Otolaryngol Head Neck Surg. 1999; 114:842-9. Failure to effectively treat CRS not only results in prolonged illness but can also result in significant complications including osteomyelitis of the facial bones, meningitis and brain abscesses. *Id.*

In clinical practice, there is a significant subpopulation of patients with CRS who remain resistant to cure despite rigorous treatment regimens including surgery, allergy therapy and prolonged antibiotic therapy. The reason for treatment failure is thought to be related to the destruction of the sinus mucociliary defense by the chronic sinus infection resulting in the development of secondary antibiotic resistant microbial colonization of the sinuses and biofilm formation. Gram-negative and Gram-positive bacteria, including *Hemophilus influenza* and *Streptococcus pneumoniae* account for 50% of clinically sampled isolates found in CRS patients. It is increasingly reported that methicillin resistant *Staphylococcus aureus* ("MRSA") and multidrug resistant *Pseudomonas aeruginosa ("MRPA")* are found in the clinical isolates of CRS patients and are a cause of antibiotic treatment failures. Numerous investigators have reported the presence of biofilms in the sinuses of patients with CRS and consider biofilm as a cause for the recalcitrant nature of persistent CRS. The presence of *Hemophilus influenza, Streptococcus pneumoniae* and *Staphylococcus aureus* biofilms have been reported to be present in patients with an unfavorable treatment outcome after aggressive antibiotic therapy and surgery for CRS. Antibiotic resistant strains of these bacteria also significantly contribute to poor clinical results with the presence of antibiotic resistant bacteria in clinical isolates as high as 30%. CRS with its chronic indolent course, resistance to antibiotics and acute exacerbations has a clinical course that parallels that of other persistent biofilm related inflammatory diseases. See Desrosiers MY, Kilty SJ. "Treatment alternatives for chronic rhinosinusitis persisting after ESS: What to do when antibiotics, steroids and surgery fail." Rhinology. 2008; 46:3-14; "Diagnosis and Management of Chronic Rhinosinusitis in Adults" cited above; Cohen M, Kofonow J, Nayak JV, et al. "Biofilms in chronic rhinosinusitis: A review." Am J Rhinol Allergy. May-June 2009; 23(3):255-60; Kilty SJ, Desrosiers MY. "Are Biofilms the Answer in the Pathophysiology and Treatment of Chronic Rhinosinusitis?" Immunol Allergy Clin N Am. 2009; 29:645-56; Foreman A, Psaltis AJ, Tan LW, Wormald P. "Characterization of bacterial and fungal biofilms in chronic rhinosinusitis." Am J Rhinol Allergy. 2009; 23(6):556-61; Hunsaker DH, Leid JG. "The relationship of biofilms to chronic rhinosinusitis." Curr Opin Otolaryngol Head Neck Surg. 16:237-41; Kilty SJ, Desrosiers MY. "The Role of Bacterial Biofilms and the Pathophysiology of Chronic Rhinosinusitis." Current Allergy and Asthma Reports. 2008; 8:227-33; Daele JJ. "Chronic sinusitis in children." Acta Otorhinolaryngol Belg. 1997; 51:285-304; Sanderson AR, Leid JG, Hunsaker D. "Bacterial Biofilms on the Sinus Mucosa of Human Subjects with Chronic Rhinosinusitis." Laryngoscope. July 2006; 116:1121-6; Cryer J, Schipor L, Perloff JR, Palmer JN. "Evidence of Bacterial Biofilms in Human Chronic Sinusitis." ORL. June 2004; 66:155-8; Ferguson BJ, Stolz, DB. "Demonstration of Biofilm in Human Bacterial Chronic Rhinosinusitis." Am J of Rhinology. September-October 2005; 19(5):452-7; Palmer JN. "Bacterial Biofilms: Do They Play a Role in Chronic Sinusitis." Otolaryngol Clin N Am. 2005; 38: 1193-1201; Harvey RJ, Lund VJ. "Biofilms and Chronic Rhinosinusitis: Systematic Review of Evidence, Current Concepts and Directions for Research." Rhinology. 2007; 45:3-13; Palmer JN. "Bacterial Biofilms in Chronic Rhinosinusitis." Ann Otol Rhinol Laryngol. 115(9 Suppl 196):35-9; Ramadan HH, Sanclement JA, Thomas JG. "Chronic Rhinosinusitis and Biofilms." Otolaryngol Head Neck Surg. 2005; 132:414-7; Sanclement JA, Webster P, Thomas J, Ramadan HH. "Bacterial Biofilms in Surgical Specimens of Patients with Chronic Rhinosinusitis." Laryngoscope. April 2005; 115:578-82; Stewart PS, Costerton JW. "Antibiotic resistance of bacteria in biofilms." Lancet. 2001 July 14; 358(9276):135-8.

Due to the failure of standard therapies to control and cure CRS, other novel non-antibiotic therapies that are able to destroy biofilms and antibiotic resistant bacteria are needed. Thus there is an urgent unmet medical need for an alternative clinically effective, non- invasive, non-toxic, cost-effective, repeatable, painless topical non-antibiotic treatment modality for CRS that improves patient outcomes and does not generate antibiotic resistance. On a more global level, the benefit of an inexpensive alternative non-antibiotic treatment for infection control would be considered a revolutionary medical advancement that would positively affect the quality of life for millions of people worldwide.

Photodynamic disinfection ("PDD") has been demonstrated to be an effective non-antibiotic antimicrobial approach *in vitro.* The use of PDD is extensively reported in the literature to be safe and effective for the photodestruction of various microorganisms. The PDD induced effect has been reported by numerous investigators to be target specific to only those organisms that have absorbed the photosensitizer and are exposed to a specific wavelength of light. Recently, PDD has been more comprehensively studied as a potential alternative to conventional antibiotic therapy as antibiotic resistant strains of bacteria become more prevalent. It has been reported in the literature that PDD is equally effective against normal strains and antibiotic resistant strains of bacteria. Furthermore, there is no evidence of bacterial photoresistance occurring after repeated PDD treatment cycles. See Usacheva MN, Teichert MC, Biel MA. "Comparison of the methylene blue and toluidine blue photobactericidal efficacy against Gram-positive and gram-negative microorganisms." Laser Surg Med. 2001; 28:1-9; Teichert MC, Jones JW, Usacheva MN, Biel MA. "Treatment of oral candidiasis with methylene blue-mediated photodynamic therapy in an immunodeficient murine model." Oral Surg Oral Med Oral Pathol Oral Radiol Endod. 2002; 93:155-60; Usacheva MN, Teichert MC, Biel MA. "The role of the methylene blue and toluidine blue monomers and dimers in the photoactivation of bacteria." J Photochem Photobiol B 2003; 71:87-98; Usacheva MN, Teichert MC, Sievert CE, Biel MA. "Effect of Ca+ on the photobactericidal efficacy of methylene blue and toluidine blue against gram-negative bacteria and the dye affinity for lipopolysaccharides." Lasers Surg Med. 2006; 38(10):946-54; Usacheva MN, Teichert MC, Sievert CE, Biel MA. "Interaction of the photobactericides methylene blue and toluidine blue with a fluorophore in Pseudomonas aeruginosa cells." Lasers Surg Med. 2008; 40(1):55-61; Jori G, Fabris C, Soncin M, et al. "Photodynamic Therapy in the Treatment of Microbial Infections: Basic Principles and Perspective Applications." Laser Surg Med. 2006; 38(5):468-81; Wong TW, Wang YY, Sheu HM, et al. "Bactericidal Effects of Toluidine Blue-Mediated Photodynamic Action on Vibrio vulnificus." Antimicrob Agents Chemother. 2005; 49(3):895-902; Millson CE, Wilson M, MacRobert AJ, et al. "Ex-vivo treatment of gastric Heliobacter infection by photodynamic therapy." J Photochem Photobiol B. 1996; 32:59-65; Soukos NS, Wilson M, Burns T, et al. "Photodynamic effects of toluidine blue on human oral keratinocytes and fibroblasts and Streptococcus sanguis evaluated in vitro." Lasers Surg Med. 1996; 18(3):253-9; Hamblin MR, Hasan T. "Photodynamic therapy: a new antimicrobial approach to infectious disease?" Photochem Photobiol Sci. 2004; 3:436-50; Wainwright M, Phoenix DA, Laycock SL, et al. "Photobactericidal activity of phenothiazinium dyes against methicillin-resistant strains of Staphylococcus aureus." FEMS Microbiol Lett. 1998; 160(2):177-81.

The photodynamic mechanism of bacterial, fungal, and/or tumor cell destruction is by perforation of the cell membrane or wall by PDD induced singlet oxygen and oxygen radicals thereby allowing the dye to be further translocated into the cell and photodamages inner organelles of the cell and induces cell death (hereinafter "photoablation"). See Harris F, Chatfield LK, Phoenix DA. "Phenothiazinium based photosensitisers - photodynamic agents with a multiplicity of cellular targets and clinical applications." Curr Drug Targets. 2005; 6(5):615-27. Importantly, the photoablation mechanism of microbial cell death is completely different from that of oral and systemic antimicrobial agents. Therefore, it is effective against antibiotic resistant bacteria. Additionally, this method of topical treatment of microbial organisms is a simple, inexpensive, nontoxic and repeatable therapy without the risk of development of antimicrobial resistance.

### SUMMARY OF THE INVENTION

The present invention provides a photosensitizer composition that uses the ethylenediaminetetraacetic acid ("EDTA") at a very low concentration (e.g., ranging from about 0.01 millimole ("mM") to about 1.25 mM) with a photosensitizer at a predetermined concentration to enhance the photoablation of the photosensitizer on inhibiting at least one targeted organism. The composition delivers enhanced photoablation compared to photoablation using the same photosensitizer alone at the same predetermined concentration. According to the invention the photosensitizer is a phenothiazine. In one embodiment, the composition further includes a pharmaceutically acceptable carrier.

The present invention also provides a photosensitizing composition for use in the photoablation of at least one targeted organism in a photoablation method comprising: applying a composition comprising a phenothiazine as photosensitizer and EDTA at a very low concentration (e.g., ranging from about 0.01 mM to about 1.25 mM) to a treatment site containing at least one targeted organism; and applying light to the treatment site at a wavelength absorbed by the photosensitizer so as to inhibit the at least one targeted organism.

### BRIEF DESCRIPTION OF THE DRAWINGS

The features and inventive aspects of the present invention will become more apparent upon reading the following detailed description, claims, and drawings, of which the following is a brief description:
FIG. 1 is a graph showing the results of the biofilm study described below in Example I;
FIG. 2 is a graph showing the results of the biofilm study described below in Example II;
FIG. 3 is a graph showing the results of *Pseudomonas aeruginosa* reduction of the biofilm study described in Example III using methylene blue at a concentration of 0.01% w/v alone and with EDTA at one of the following concentrations: 0.25 mM, 0.5 mM, 0.75 mM and 1.25 mM;
FIG. 4 is a graph showing the results of *Pseudomonas aeruginosa* reduction of the biofilm study described in Example III using methylene blue at a concentration of 0.03% w/v alone and with EDTA at one of the following concentrations: 0.25 mM, 0.5 mM, 0.75 mM and 1.25 mM;
FIG. 5 is a graph showing the results of *Pseudomonas aeruginosa* reduction of the biofilm study described in Example III using methylene blue at a concentration of 0.05% w/v alone and with EDTA at one of the following concentrations: 0.25 mM, 0.5 mM, 0.75 mM and 1.25 mM;
FIG. 6 is a graph showing the results of MRSA and MRPA reduction of the planktonic study described in Example IV using methylene blue at a concentration of 0.005% w/v alone and with EDTA at one of the following concentrations: 0.005 mM, 0.01 mM, 0.025 mM, 0.05 mM, 0.075 mM, 0.1 mM 0.175 mM, 0.25 mM and 0.4mM without any light treatment; and
FIG. 7 is a graph showing the results of MRSA and MRPA reduction of the planktonic study described in Example IV using methylene blue at a concentration of 0.005% w/v alone and with EDTA at one of the following concentrations: 0.005 mM, 0.01 mM, 0.025 mM, 0.05 mM, 0.075 mM, 0.1 mM 0.175 mM, 0.25 mM and 0.4mM with light treatment.

### DETAILED DESCRIPTION OF THE INVENTION

### 1. The Composition

The composition of the present invention includes a phenothiazine as photosensitizer
and EDTA at a very low concentration (e.g., ranging from about 0.01 mM (0.00029 % w/v) to about 1.25 mM (0.03653 % w/v)). As shown in the Examples below, the composition provides greater or enhanced photoablation against at least one targeted organism as compared to the amount of photoablation against the same targeted organism using the photosensitizer alone (under similar or even identical parameters) ("enhanced photoablation"). The at least one targeted organism can be microbe(s), fungal cell(s), virus, tumor cell(s), or a combination thereof. For example, the Examples described below show that the composition provides enhanced photoablation to (i) Gram-negative bacterial cells (e.g., MRPA, *Pseudomonas aeruginosa,* or the like); and (ii) a combination of Gram-negative bacteria; cells (e.g., MRPA, *Pseudomonas aeruginosa,* or the like) and Gram-positive bacterial cells (e.g., MRSA or the like). Moreover, the Examples below also show that the composition provides enhanced photoablation to both planktonic bacteria cells and biofilm bacteria cells.

EDTA has been approved by the FDA as a preservative in packaged foods, vitamins, and baby food and has low toxicity. In medicine, using much higher concentrations than the concentrations used in the present invention, EDTA is used in chelation therapy for acute hypercalcemia, mercury poisoning and lead poisoning. See Inactive ingredient guide. U.S. Department of Health and Human Services, Public Health Services, Food and Drug Administration, Rockville, MD, 1996; Handbook of Pharmaceutical Excipients, 3rd ed., AH Kibbe (ed.) (London: Pharmaceutical Press, 2000), 33-5.

The present invention uses EDTA at a very low concentrations (e.g., ranging from about 0.01 mM to about 1.25 mM) to enhance photoablation of the at least one targeted organism by the photosensitizer of the composition. Exemplary concentrations of EDTA suitable for the composition are from about 0.01 mM to about 1.25 mM; from about 0.01 mM to about 1 mM (0.029224 % w/v); from about 0.01mM to about 0.75 mM (0.021918 % w/v); from about 0.025 mM (0.0007306 % w/v) to about 1.25 mM; from about 0.025 mM to about 1 mM; from about 0.025 to about 0.75 mM; from more than about 0.01 mM to about 1.25 mM; from more than about 0.005 mM (0.00014612 % w/v) to about 1.25 mM; from about 0.01 mM to about 0.175 mM (0.0051142 % w/v); from more than about 0.005 mM to less than about 0.25 mM (0.007306 % w/v); and from about 0.01 mM to less than about 0.25 mM.

Since the EDTA concentration of the composition of the present invention is very low, it is believed that the enhanced photoablation provided by the composition is likely caused by the electron transfer between EDTA and the photosensitizer especially when the photosensitizer is in the triplet-excited state (i.e., during the time when the photosensitizer is activated by light). For example, during the electron transfer from EDTA to a photosensitizer such as methylene blue, a large number of free radicals with significant quantum yields are generated. See Pal MK, Manna PCH. "Effect of DNA and other polyanions on the EDTA induced photoreduction of thionine." Makromol Chem. 1982; 183:2811-21; Bonneau R, Joussot-Dubien J, Faure J. "Mechanism of photoreduction of thiazine dyes by EDTA studied by flash photolysis." I Photochem Photobiol. 1973; 17:313-9; Pal MK, Mazumdar KK. "Photoreduction of dyes catalysed by organic and bio-molecules." Histochemistry. 1974; 40:267-74. EDTA is an effective chelating agent that could form complexes (via four carboxylate and two amino groups) with a photosensitizer. It is believed that EDTA formed complex with the photosensitizer and provides electron transfer to the photosensitizer. This electron transfer enhances the photoablation ability of the light activated photosensitizer against the at least one targeted organism such as microbe(s). This same mechanism can also enhance the photosensitizer's ability to inhibit tumor cells during photoablation.

It is believed that the electron transfer is not dependent upon a specific type of photosensitizer as long as the photosensitizer can be light activated. The photosensitizer of the present invention is a phenothiazine (e.g., methylene blue, toluidine blue O and their derivatives, etc.).

Depending on the desired application, the composition may optionally comprise a plurality of the photosensitizers. The amount or concentration of the photosensitizer(s) may vary depending upon the desired application, the particular photosensitizer(s) used, and the at least one targeted organism to be inhibited. The term inhibit and/or inhibited shall mean prevent, reduce, destroy, kill, eliminate, or the like. For example, concentration of the photosensitizer(s) in the composition may range from about 0.0001% w/v to about 25% w/v, from about 0.001% w/v to about 10% w/v, from about 0.01% w/v to about 1% w/v, from about 0.01% w/v to about 0.1% w/v, from about 0.005% w/v to about 0.05% w/v, from about 0.01% w/v to about 0.5 % w/v, from about 0.03 % w/v to about 0.05 % w/v, from about 0.1 % w/v to about 0.5% w/v.

The term "about" as used herein in this specification shall mean +/-5% of the stated value.

EDTA may weaken the lipopolysaccharide ("LPS") barrier in the outer membrane of Gram-negative bacteria replacing the cations of Ca²⁺ and Mg²⁺ and improve the photosensitizer penetration through the Gram-negative bacteria membrane (US2009/093470 A1).

However, due to the fact that the composition of the present invention provides a very low concentration of EDTA, it is believed that this membrane penetration mechanism is unlikely to be responsible for the enhanced photoablation provided by the composition.

In a preferred embodiment of the present invention, the photosensitizer is methylene blue. Methylene blue is a phenothiazinium salt that has a strong absorption at wavelengths longer than about 620 nm. The absorbance peak of methylene is at about 664 nm and its optical extinction coefficient is 81600 M⁻¹cm⁻¹. The photoactivity of methylene blue results in two types of photooxidations: (i) the direct reaction between the photoexcited dye and substrate by hydrogen abstraction or electron transfer creating different active radical products; and (ii) the direct reaction between the photoexcited dye in triplet state and molecular oxygen producing singlet oxygen. Both kinds of active generated products are strong oxidizers and cause cellular damage, membrane lysis and protein inactivation. Methylene blue has a high quantum yield of the triplet state formation (∼T=0.52-0.58) and a high yield of the singlet oxygen generation (0.2 at pH 5 and 0.94 at pH 9). See Eimer M, Tuite JM, Kelly J. "Properties of various dyes." Photochem Photobiol. 1993; 321:103; McVae J. "Optical properties of natural dyes." Chem Soc Trans. 2; 1870:1778.

The composition can further optionally include a pharmaceutically acceptable carrier. The pharmaceutically acceptable carrier is a diluent, adjuvant, excipient, or vehicle with which the other components (e.g., the photosensitizer and the EDTA, etc.) of the composition are administered. The pharmaceutically acceptable carrier is preferably approved by a regulatory agency of the Federal or a state government, or listed in the U.S. Pharmacopeia or other generally recognized pharmacopeia for use in animals, and more particularly in humans. The pharmaceutically acceptable carriers are preferably sterile liquids. Examples of the pharmaceutically acceptable carriers include but are not limited to water, saline solution, dextrose solution, glycerol solution, phosphate buffered saline solution, alcohol (e.g., ethanol or the like), and other solvents such as propylene glycol etc.

The composition may optionally comprise addition components such as anti-inflammatory agents, buffers, salts for adjusting the tonicity of the solution, antioxidants, additional preservatives, viscosity-altering agents (carboxymethylcellulose, hydroxypropylmethylcellulose, hydroxypropylcellulose), flavoring/scent, oxygen carrier molecules, cell permeabilizing agents, antibiotics, bactericides/bacteriostats or the like.

### 2. The Method

The present invention provides a photosensitizing composition for use in a method for photoablation
comprising: applying the composition of the present invention described above to a treatment site containing at least one targeted organism; and applying light to the treatment site at a wavelength absorbed by the photosensitizer so as to inhibit the at least one targeted organism. As discussed above, the at least one targeted organism can be any of the following: microbe(s), fungal cell(s), virus, tumor cell(s), or a combination thereof. The treatment site can be any area where photoablation is desired (e.g., around human tissue, animal tissue, another substrate or otherwise).

The present invention provides a photosensitizing composition for use in a method of photodynamic
disinfection of a treatment site comprising: applying the composition of the present invention described above to a treatment site and applying light to the treatment site at a wavelength absorbed by the photosensitizer so as to inhibit at least one microbe located within the treatment site.

Microbes can include any and all disease-related microbes such as virus, fungus, and bacteria including Gram-negative organisms, Gram-positive organisms or the like. Some examples of microbes include but are not limited to, of *Hemophilus influenza, Streptococcus pneumonia, Staphylococcus aureus, Methicillin-resistant Staphylococcus aureus* ("MRSA"), *Escherichia coli, Enterococcus fecalis, multidrug resistant Pseudomonas aeruginosa ("MRPA"), Pseudomonas aeruginosa, Aspergillus, Candida, Clostridium difficile, Staphylococcus epidermidis, Acinobacter sp*., etc.

The wavelength can be any wavelength(s) of light that can be absorbed by the photosensitizer(s) of the composition. The wavelengths include wavelengths selected from the continuous electromagnetic spectrum such as ultraviolet ("UV"), visible, the infrared (near, mid and far), etc. For examples, the wavelengths are between about 160 nm to about 1600 nm, between 400 nm to about 800 nm, between about 500 nm to about 850 nm, between about 600 nm to about 700 nm although the wavelengths may vary depending upon the particular photosensitizer(s) used and the light intensity. The light may be produced by any suitable art-disclosed light emitting devices for use in photodynamic disinfection such as lasers (e.g., non-thermal lasers or the like), light emitting diodes ("LEDs"), incandescent sources, fluorescent sources, or the like.

Depending on the photosensitizer concentration and the power of the light emitting device(s), the application of light to the treatment site may only require a short period of time such as from about 15 seconds to less than about 5 minutes, preferably from about 15 seconds to about two minutes, more preferably for about 15 seconds to about 90 seconds, and most preferably for about 30 seconds to 60 seconds. Alternatively, the application of light to treatment site may require a longer period of time such as from about 1 minute to about 20 minutes. The light energy provided during each cycle of application of light may range from about 1 J/cm² to about 75 J/cm², from about 1 J/cm² to about 72 J/cm², from 1 J/cm² to about 50 J/cm², from 2 J/cm² to about 45 J/cm², from about 1 J/cm² to about 25 J/cm², from about 5 J/cm² to about 20 J/cm², and from at about 6 J/cm² to about 12 J/cm². Depending on the nature and extent of the microbes located at the treatment site, the practitioner may apply multiple cycles of light applications (e.g., about 2 to about 10, about 3 to about 5, etc.) to the treatment site thereby resulting in a total accumulated light energy applied to treatment site that can be substantially higher than the light energy provided during each cycle. For example, the total accumulated light energy dose may range from about 1 J/cm² to about 200 J/cm², from about 5 J/cm² to about 200 J/cm², from about 10 J/cm² to about 200 J/cm², about 1 J/cm² to about 175 J/cm², from about 5 J/cm² to about 175 J/cm², from about 10 J/cm² to about 175 J/cm², about 1 J/cm² to about 150 J/cm², from about 5 J/cm² to about 150 J/cm², from about 10 J/cm² to about 150 J/cm², about 1 J/cm² to about 100 J/cm², from about 5 J/cm² to about 100 J/cm², from about 10 J/cm² to about 100 J/cm², about 1 J/cm² to about 72 J/cm², from about 5 J/cm² to about 72 J/cm², from about 10 J/cm² to about 72 J/cm².

Again depending on the nature and extent of the targeted organism(s) located at the treatment site, the entire method and/or the light application step can be repeated multiple times (e.g., about 2 to about 10, about 3 to about 5, etc.) until the desired effects have been reached. It is preferred that the selections of photosensitizer concentration, wavelength, and/or total accumulated light energy applied to treatment site will allow the method of the present invention to reduce over about 90%, more preferably over 95%, and most preferably over 99% of the target microbes located at the treatment site. It is also preferred that the application of light to the treatment site does not cause physiological damage to the host tissues at and/or surround the treatment site.

The composition and method of the present invention discussed above can be used to treat any disease caused by the at least one targeted organism. For example and without limitation, they can be used to treat any of the numerous diseases caused by Gram-negative bacteria such as sinusitis, CRS, pneumonia and bronchitis (especially in cystic fibrosis), otitis externa, burn infections, urinary tract infections, colon infections, uterus infections, osteomyelitis, chronic bronchitis, etc. Moreover, the composition and method of the present invention can also be used to inhibit tumor cells. Accordingly, the present invention includes a photosensitizing composition for use in a method for treating cancer comprising: applying the composition of the present invention described above to the treatment site; and applying light to the treatment site at a wavelength absorbed by the photosensitizer so as to inhibit at least one tumor cell located within the treatment site.

The present invention is not being limited in scope by the specific embodiments described herein. Indeed, various modifications of the invention in addition to those described herein will become apparent to those skilled in the art from the foregoing description and the accompanying figures. It is further to be understood that all numerical values are approximate and are provided for description only.

The following examples provided in accordance to the present invention are for illustrative purpose only and are not intended as being exhaustive or limiting of the invention.

### Example I

An *in vitro* study was conducted on polymicrobial biofilms containing MRPA and MRSA using the following compositions: (i) compositions each comprising EDTA at one of the following concentrations: 0.5 mM (0.014612 % w/v), 0.75 mM, 1.0 mM and 1.25 mM; (ii) composition comprising of methylene blue at a concentration of 0.05 % w/v; (iii) compositions each comprising methylene blue at a concentration of 0.05 % w/v and EDTA at one of the following concentrations: 0.5 mM, 0.75 mM, 1.0 mM and 1.25 mM. Clinical isolates of MRPA and MRSA were used in this study. Overnight tryptic soy broth (Remel, Lenexa, KS) cultures were centrifuged for 15 minutes at 5,000 RPM. The supernatants were removed and the cells washed and resuspended in 1x phosphate buffered saline (PBS) by alternate rounds of centrifugation for 2 minutes at 12.1 xg and resuspension. Resulting bacterial suspensions were diluted with PBS to obtain approximately 1.5x10⁸ cells/mL of each bacterium measured using a CrystalSpec nephlometer (BD Diagnostic Systems, Sparks, MD). The suspensions were then combined. The FC 270 dual-flow cell system (BioSurface Technologies Corp., Bozeman, MT) was assembled as described and validated by Leung et al and others. See Leung KP, Crowe TD, Abercrombie JJ, et al. "Control of oral biofilm formation by an antimicrobial decapeptide." J Dent Res. 2005; 84(12):1172-7; Singh PK, Parsek MR, Greenberg EP, Welsh MJ. "A comparison of innate immunity prevents biofilm development." Nature. 2002; 417:552-5.

Biofilms were grown on 5.5x7.5x0.1 cm silicone sheeting (Invotec International, Jacksonville, FL) cut into circular 1.27 cm discs. Flow cells were inoculated with 500 µL of the bacterial suspension and the bacteria were allowed to adhere to the disc surfaces for one hour. Next, tryptic soy broth was pumped through the flow cells at a constant rate of 30 mL h⁻¹ for 24 hours using a peristaltic pump (Control Company, Friendswood, TX). The silicone discs were removed from the flow cells and gently washed thrice with PBS to remove non-adherent bacteria resulting in the biofilm discs to be used in this study.

Next, each of the biofilm discs was placed into an empty well of a tissue culture plate. Except for the biofilm discs that served as the control, all other biofilm discs were each treated with 90 µL of one of the solutions described below for 5 minutes in the dark.

Methylene blue solution was prepared using methylene blue at 99.8% purification (Sigma-Aldrich, St. Louis, MO) and dissolving it into USP water (i.e., water that meets meeting United States Pharmacopeia specifications specifications). The above-described compositions were prepared and adjusted to a pH of about 6.3 using 1 Normal ("N") hydrochloric acid ("HCl") and/or 1 N sodium hydroxide ("NaOH").

After the dark incubation, all of the treated biofilm discs were removed, gently washed with PBS, and placed into different plate wells. Some of the treated biofilm discs were exposed to 664 nm light using a non-laser light at a power density of 150 mW/cm² and a light energy dose of 45 J/cm².

Thereafter, all of the biofilm discs were each placed in a sterile 50 mL centrifuge tube with 1 mL of PBS, vortexed vigorously for one minute. Samples for culture were serially diluted in PBS and spirally plated using an Autoplate 4000 (Spiral Biotech, Norwood, MA) onto tryptic soy agar with 5% sheep's blood (Remel). Plates were incubated at 37°C for 24 hours. The colonies were counted by a Protos automated counter (Synoptics, Frederick, MD) to determine the total number of viable bacteria and expressed as colony forming units (CFU). This study was repeated numerous times using the same PDD equipment and methodology (e.g., obtaining at least 14 to 28 samples of each type of test conditions).

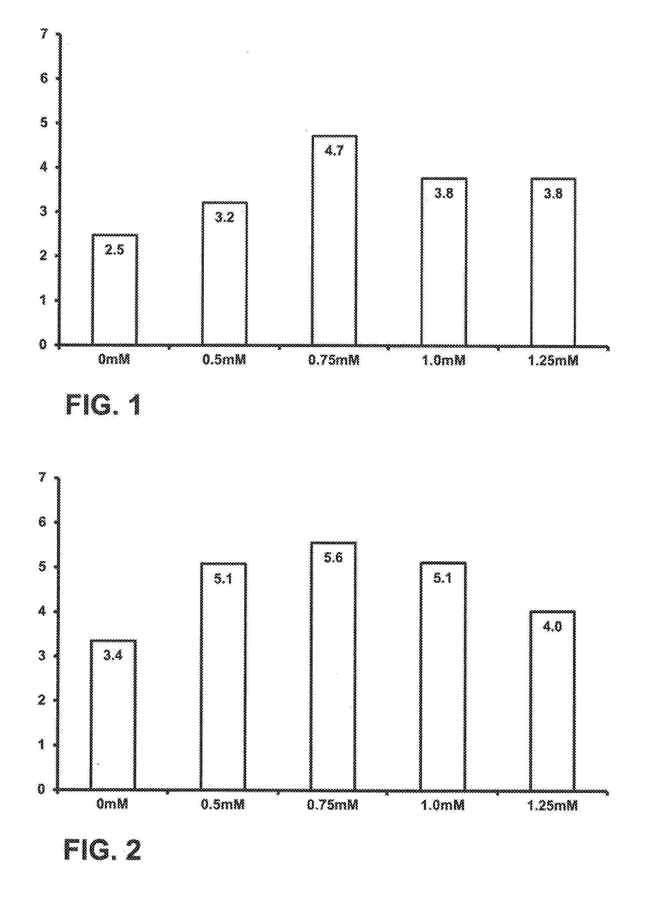

The results of the study as described above of applying PDD on polymicrobial biofilm discs using the above-described compositions are described in FIG. 1. The vertical axis of FIG. 1 shows the bacterial reduction in log₁₀ CFU/ml when compared to the control (i.e., samples of bacteria which that did not receive any treatment such as light, EDTA and/or methylene blue, or the like). The horizontal axis shows the concentrations of EDTA for each of the compositions used for PDD treatment. For example, the first column shows the composition used contained methylene blue at a concentration of 0.05 % w/v with no EDTA added provided a 2.5 log₁₀ reduction compared to the control; the second column shows the composition used contained methylene blue at a concentration of 0.05 % w/v and 0.5 mM EDTA provided a 3.2 log₁₀ reduction compared to the control; the third column shows the composition used contained methylene blue at a concentration of 0.05 % w/v and 0.75 mM EDTA provided a 4.7 log₁₀ reduction compared to the control; the fourth column shows the composition used contained methylene blue at a concentration of 0.05 % w/v and 1.0 mM EDTA provided a 3.8 log₁₀ reduction compared to the control; and the fifth column shows the composition used contained methylene blue at a concentration of 0.05 % w/v and 1.25 mM EDTA provided a 3.8 log₁₀ reduction compared to the control.

The results shown in FIG. 1 demonstrate that PDD using the a composition containing a photosensitizer such as methylene blue and EDTA at a very low concentration (e.g., from 0.5 mM to 1.25 mM) had significantly more effective/enhanced photoablation compared to using a composition containing the photosensitizer alone against biofilm microbes such as MRPA and MRSA. The study also importantly demonstrates a dose response with concentrations of EDTA. For example, the study shows that the most effective concentrations of EDTA used with methylene blue at the concentration of 0.05 % w/v in the study were 0.75 mM.

The study also found that EDTA alone did not result in any statistically significant biofilm bacterial count reduction. None of the biofilm discs treated with EDTA at various concentrations (i.e., 0.5 mM, 0.75 mM, 1.0 mM and 1.25 mM) showed any statistically significant biofilm bacterial count reduction.

### Example II

Another *in vitro* study of polymicrobial biofilms containing MRPA and MRSA was conducted using the compositions and protocol described in Example I. The results of this study are described in FIG. 2. The vertical axis of FIG. 2 shows the bacterial reduction in log₁₀ CFU/ml when compared to the control. The horizontal axis shows the concentrations of EDTA for each of the compositions used for PDD treatment. For example, the first column shows the composition used contained methylene blue at a concentration of 0.05 % w/v with no EDTA added provided a 3.4 log₁₀ reduction compared to the control; the second column shows the composition used contained methylene blue at a concentration of 0.05 % w/v and 0.5 mM EDTA provided a 5.1 log₁₀ reduction compared to the control; the third column shows the composition used contained methylene blue at a concentration of 0.05 % w/v and 0.75 mM EDTA provided a 5.6 log₁₀ reduction compared to the control; the fourth column shows the composition used contained methylene blue at a concentration of 0.05 % w/v and 1.0 mM EDTA provided a 5.1 log₁₀ reduction compared to the control; and the fifth column shows the composition used contained methylene blue at a concentration of 0.05 % w/v and 1.25 mM EDTA provided a 4.0 log₁₀ reduction compared to the control.

The results shown in FIG. 2 further support the findings discussed above in Example I in that PDD using the compositions containing a photosensitizer and EDTA at a very low concentration (e.g., from 0.5 mM to 1.25 mM) had significantly more effective/enhanced photoablation compared to the photosensitizer alone.

### Example III

An *in vitro* study was conducted on polymicrobial biofilms containing MRSA and *Pseudomonas aeruginosa.* Stock cultures of MRSA ATCC # 33592 and *Pseudomonas aeruginosa* ATCC # 9027 were grown aerobically overnight on tryptic soy agar, harvested individually, and suspended in PBS solution with MRSA at a concentration of ∼2x10⁸ CFU/ml and *Pseudomonas aeruginosa* at a concentration of ∼3x10⁸ CFU/ml. Cell densities were measured as a function of absorbance at 420 nm using a Genesys 10 spectrophotometer (Thermo Scientific, Pittsburgh, PA). *Pseudomonas aeruginosa* inocula were then serial diluted in PBS to a concentration of ∼3x10⁴ CFU/ml. The two suspended inocula (MRSA and *Pseudomonas aeruginosa*) were mixed at a ratio of 1:1 and then further diluted 1:40 into tryptic soy broth to achieve the biofilm inoculum of having a MRSA final concentration of ∼2.5x10⁶ CFU/ml and *Pseudomonas aeruginosa* at a final concentration of ∼3.75x10³ CFU/ml

The biofilm inoculum, prepared as described above, was then pipetted onto 8 mm diameter sterile silicone discs placed inside a 24-well culture plate with 600 µl of the biofilm inoculum per well. The plates were then allowed to shake for 24 hours at 125 rotations per minute using an Innova 200 platform gyrorotary shaker (New Brunswick Scientific Co, Edison NJ) at 35°C. After 24 hours, all residual liquid from each well was removed and 600 µl of sterile tryptic soy broth was added to each well. Thereafter, the plates were again allowed to shake for another 24 hours using the same shaker as described above. After the 48 hour total biofilm growth period, residual liquids were removed by rinsing each disc two times in PBS resulting in the biofilm discs for use in this study.

After the biofilm discs have been prepared as discussed above, each biofilm disc then received one of the following treatment: (i) treatment with 20 µl of PBS alone ("control"); (ii) treatment with a composition in the amount of 20 µl containing (a) methylene blue at one of the following concentrations: 0.01 % w/v, 0.03 % w/v, or 0.05% w/v, and (b) USP water; and (iii) treatment with a composition in the amount of 20 µl containing (a) methylene blue at one of the following concentrations: 0.01 % w/v, 0.03 % w/v, or 0.05% w/v, (b) EDTA at one of the following concentrations 0.25mM, 0.5 mM, 0.75 mM, and 1.25 mM, (c) 5 % w/v ethanol ("ETOH") and (d) USP water. A glass cover slip was applied to the silicone disc. Each of the above-described compositions was allowed to incubate on its respective biofilm disc for 3.5 minutes. Thereafter, all biofilm discs except for the control were then exposed to 669 nm non-thermal laser light at a power density of 150 mW/cm² for a period of 8 minutes resulting in a total light energy dose of 72 J/cm².

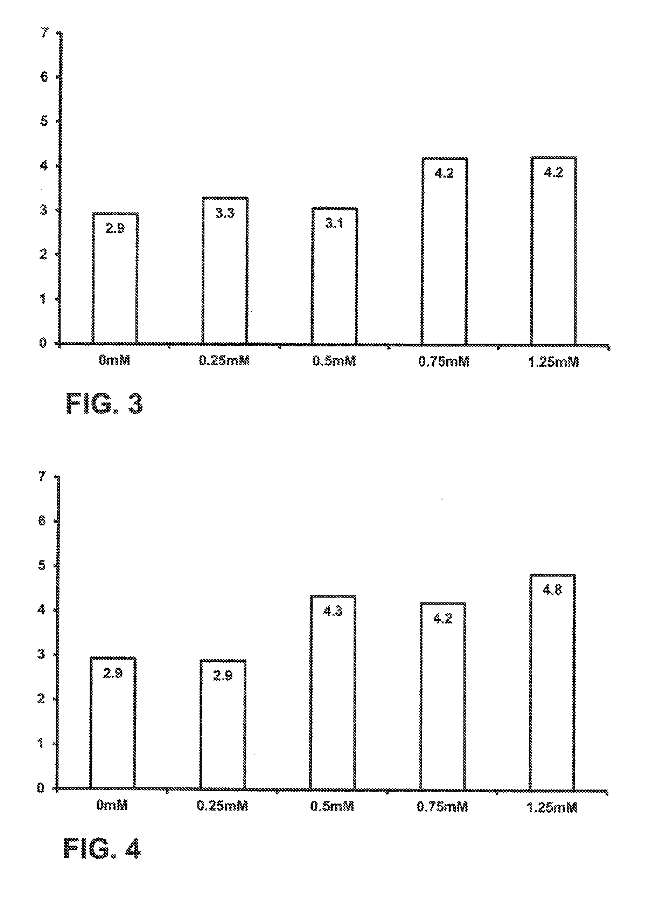

Immediately following illumination, the glass cover slip was removed carefully using sterile forceps and biofilm disc surfaces were swabbed using a calcium alginate swab following a reproducible "X" pattern. Swabs were placed in 0.5 ml recovery media (0.5% Tween-80® in PBS) and sonicated for 15 minutes using a model 250HT sonicator (VWR, West Chester, PA). Serial dilutions were performed and samples in the amount of 100 µl per sample were plated on onto tryptic soy agar (Remel). The tryptic soy agar plates were incubated at 37°C for 48 hours before assessing viable colony counts. Colony counts were counted separately for each bacterial species (i.e., MRSA and *Pseudomonas aeruginosa*) providing differentiation of reduction for each of the bacterial species. Counts for all experimental and control conditions were averaged and expressed as CFU/ml. The bacteria reduction rate was calculated as surviving CFU/ml in experimental conditions vs. the control expressed as a log₁₀ reduction from the control. Light-alone test conditions were also performed on biofilm discs which confirmed the results provided in this study as shown in FIGS. 3-5 was not the result of any potential thermal killing of bacteria. All experimental and control conditions were run in triplicate. The results of this biofilm study show statistically significant reduction of *Pseudomonas aeruginosa* bacterial count as shown in FIGS. 3-5 but no statistically significant reduction of MRSA bacterial count for any of the compositions used.

The results shown in FIGS. 3-5 demonstrate that PDD using the a composition containing a photosensitizer such as methylene blue and EDTA at a very low concentration (e.g., from 0.25 mM to 1.25 mM) had significantly more effective/enhanced photoablation compared to using a composition containing the photosensitizer alone against biofilm *Pseudomonas aeruginosa* bacterial count. The vertical axis of FIGS. 3-5 shows the bacterial reduction in log₁₀ CFU/ml when compared to the control samples of biofilm *Pseudomonas aeruginosa* which that did not receive any PDD treatment. The horizontal axis of FIGS. 3-5 shows the concentrations of EDTA for each of the methylene blue compositions used for PDD treatment.

FIG. 3 shows the results of *Pseudomonas aeruginosa* reduction of this biofilm study using methylene blue at a concentration of 0.01% w/v alone and with EDTA at one of the following concentrations: 0.25 mM, 0.5 mM, 0.75 mM and 1.25 mM. In FIG. 3, the first column shows the composition used contained methylene blue at a concentration of 0.01 % w/v with no EDTA added provided a 2.9 log₁₀ reduction compared to the control; the second column shows the composition used contained methylene blue at a concentration of 0.01 % w/v and 0.25 mM EDTA provided a 3.3 log₁₀ reduction compared to the control; the third column shows the composition used contained methylene blue at a concentration of 0.01 % w/v and 0.5 mM EDTA provided a 3.1 log₁₀ reduction compared to the control; the fourth column shows the composition used contained methylene blue at a concentration of 0.01 % w/v and 0.75 mM EDTA provided a 4.2 log₁₀ reduction compared to the control; and the fifth column shows the composition used contained methylene blue at a concentration of 0.01 % w/v and 1.25 mM EDTA provided a 4.2 log₁₀ reduction compared to the control.

FIG. 4 shows the results of *Pseudomonas aeruginosa* reduction of this biofilm study using methylene blue at a concentration of 0.03% w/v alone and with EDTA at one of the following concentrations: 0.25 mM, 0.5 mM, 0.75 mM and 1.25 mM. In FIG. 4, the first column shows the composition used contained methylene blue at a concentration of 0.03 % w/v with no EDTA added provided a 2.9 log₁₀ reduction compared to the control; the second column shows the composition used contained methylene blue at a concentration of 0.03% w/v and 0.25 mM EDTA provided a 2.9 log₁₀ reduction compared to the control; the third column shows the composition used contained methylene blue at a concentration of 0.03 % w/v and 0.5 mM EDTA provided a 4.3 log₁₀ reduction compared to the control; the fourth column shows the composition used contained methylene blue at a concentration of 0.03 % w/v and 0.75 mM EDTA provided a 4.2 log₁₀ reduction compared to the control; and the fifth column shows the composition used contained methylene blue at a concentration of 0.03 % w/v and 1.25 mM EDTA provided a 4.8 log₁₀ reduction compared to the control.

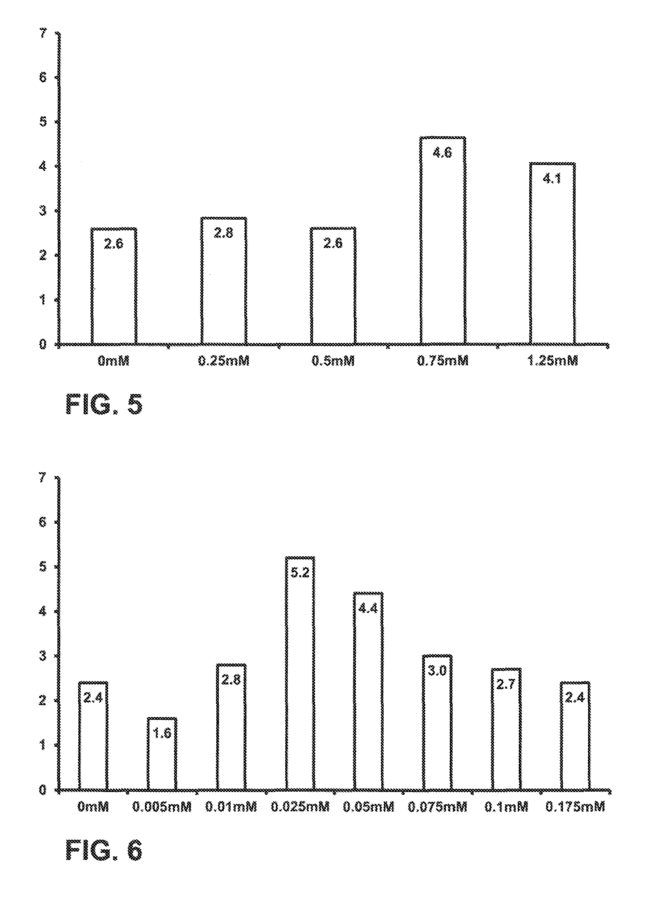

FIG. 5 shows the results of *Pseudomonas aeruginosa* reduction of this biofilm study using methylene blue at a concentration of 0.05% w/v alone and with EDTA at one of the following concentrations: 0.25 mM, 0.5 mM, 0.75 mM and 1.25 mM. In FIG. 5, the first column shows the composition used contained methylene blue at a concentration of 0.05 % w/v with no EDTA added provided a 2.6 log₁₀ reduction compared to the control; the second column shows the composition used contained methylene blue at a concentration of 0.05% w/v and 0.25 mM EDTA provided a 2.8 log₁₀ reduction compared to the control; the third column shows the composition used contained methylene blue at a concentration of 0.05 % w/v and 0.5 mM EDTA provided a 2.6 log₁₀ reduction compared to the control; the fourth column shows the composition used contained methylene blue at a concentration of 0.05 % w/v and 0.75 mM EDTA provided a 4.6 log₁₀ reduction compared to the
control; and the fifth column shows the composition used contained methylene blue at a concentration of 0.05 % w/v and 1.25 mM EDTA provided a 4.1 log₁₀ reduction compared to the control.

The results shown in FIGS. 3-5 demonstrate that PDD using the a composition containing a photosensitizer such as methylene blue and EDTA at a very low concentration (e.g., from 0.25 mM to 1.25 mM) had significantly more effective/enhanced photoablation compared to using a composition containing the photosensitizer alone against polymicrobial biofilms containing *Pseudomonas aeruginosa.*

### Example IV

An *in vitro* study was conducted on planktonic MRPA and MRSA using the following compositions. (i) compositions each comprising EDTA at one of the following concentrations: 0.005 mM, 0.01 mM, 0.025 mM, 0.05 mM (0.0014612 % w/v), 0.075 mM (0.0021918 % w/v), 0.1 mM, 0.175 mM, 0.25 mM and 0.4mM (0.0116896 % w/v); (ii) composition comprising of methylene blue at a concentration of 0.005 % w/v; and (iii) compositions each comprising of methylene blue at a concentration of 0.005 % w/v with EDTA at one of the following concentrations: 0.005 mM, 0.01 mM, 0.025 mM, 0.05 mM, 0.075 mM, 0.1 mM 0.175 mM, 0.25 mM and 0.4mM. The planktonic MRPA and MRSA samples were prepared in the same fashion as described in Example I in order to obtain approximately 1.5x10⁸ cells/mL of MRPA and approximately 1.5x10⁸ cells/mL of MRSA which were then combined.

Methylene blue solution was prepared using methylene blue at 99.8% purification (Sigma-Aldrich, St. Louis, MO) and dissolving it into USP water. Each of the compositions discussed above was prepared and then mixed with the combined bacterial solution of MRPA and MRSA and incubated for 30 seconds ("treated samples"). Thereafter, test tubes were prepared each containing 50 µl of one of the treated samples. Some of these test tubes were kept in dark incubation for 400 seconds while the remaining test tubes were exposed to 664 nm light using a non-laser light at a power density of 100 mW/cm² and a light energy dose of 10 J/cm².

Thereafter, a cotton-tipped swab (Puritan, Guilford, ME) was dipped into the suspension contained within each test tube, placed in 1 mL of PBS with the stick broken off, and then vortexed for 3-5 seconds ("test samples"). These test samples were serially diluted in PBS and spirally plated using an Autoplate 4000 (Spiral Biotech, Norwood, MA) onto tryptic soy agar. Plates were incubated at 37°C for 24 hours. The colonies were counted by a Protos automated counter (Synoptics, Frederick, MD) to determine the number of viable bacteria and expressed as colony forming units (CFU). All experimental and control conditions were repeated numerous times using the same PDD equipment and methodology. Counts for all experimental and control conditions were averaged and expressed as CFU/ml.

The planktonic MRPA and MRSA reduction rate was calculated as surviving CFU/ml in experimental conditions vs. the control (i.e., test samples that received no treatment) expressed as a log₁₀ reduction from control and shown in FIGS. 6-7.

FIG. 6 shows the results of the planktonic MRPA and MRSA reduction treated with the following compositions without any light (i.e., dark incubation for 400 seconds): composition comprising of methylene blue at a concentration of 0.005 % w/v alone, and compositions each comprising of methylene blue at a concentration of 0.005 % w/v with EDTA at one of the following concentrations: 0.005 mM, 0.01 mM, 0.025 mM, 0.05 mM, 0.075 mM, 0.1 mM 0.175 mM, 0.25 mM and 0.4mM. The vertical axis of FIG. 6 shows the bacterial reduction in log₁₀ CFU/ml when compared to the control. The horizontal axis shows the concentrations of EDTA for each of the compositions used. For example, the first column shows the composition used contained methylene blue at a concentration of 0.005 % w/v with no EDTA added provided a 2.4 log₁₀ reduction compared to the control; the second column shows the composition used contained methylene blue at a concentration of 0.005 % w/v and 0.005 mM EDTA provided a 1.6 log₁₀ reduction compared to the control; the third column shows the composition used contained methylene blue at a concentration of 0.005 % w/v and 0.01 mM EDTA provided a 2.8 log₁₀ reduction compared to the control; the fourth column shows the composition used contained methylene blue at a concentration of 0.005 % w/v and 0.025 mM EDTA provided a 5.2 log₁₀ reduction compared to the control; the fifth column shows the composition used contained methylene blue at a concentration of 0.005 % w/v and 0.05 mM EDTA provided a 4.4 log₁₀ reduction compared to the control; the sixth column shows the composition used contained methylene blue at a concentration of 0.005 % w/v and 0.075 mM EDTA provided a 3.0 log₁₀ reduction compared to the control; the seventh column shows the composition used contained methylene blue at a concentration of 0.005 % w/v and 0.1 mM EDTA provided a 2.7 log₁₀ reduction compared to the control; the eight column shows the composition used contained methylene blue at a concentration of 0.005 % w/v and 0.175 mM EDTA provided a 2.4 log₁₀ reduction compared to the control.

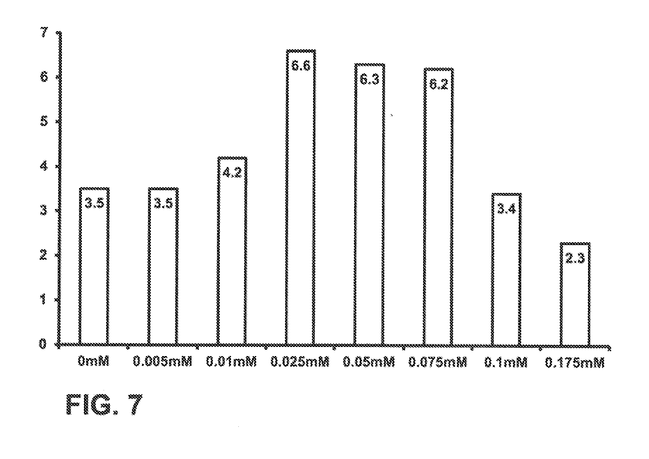

FIG. 7 shows the results of the planktonic MRPA and MRSA reduction treated with the following compositions with light treatment: composition comprising of methylene blue at a concentration of 0.005 % w/v alone, and compositions each comprising of methylene blue at a concentration of 0.005 % w/v with EDTA at one of the following concentrations: 0.005 mM, 0.01 mM, 0.025 mM, 0.05 mM, 0.075 mM, 0.1 mM 0.175 mM, 0.25 mM and 0.4mM. The vertical axis of FIG. 7 shows the bacterial reduction in log₁₀ CFU/ml when compared to the control. The horizontal axis shows the concentrations of EDTA for each of the compositions used. For example, the first column shows the composition used contained methylene blue at a concentration of 0.005 % w/v with no EDTA added provided a 3.5 log₁₀ reduction compared to the control; the second column shows the composition used contained methylene blue at a concentration of 0.005 % w/v and 0.005 mM EDTA provided a 3.5 log₁₀ reduction compared to the control; the third column shows the composition used contained methylene blue at a concentration of 0.005 % w/v and 0.01 mM EDTA provided a 4.2 log₁₀ reduction compared to the control; the fourth column shows the composition used contained methylene blue at a concentration of 0.005 % w/v and 0.025 mM EDTA provided a 6.6 log₁₀ reduction compared to the control; the fifth column shows the composition used contained methylene blue at a concentration of 0.005 % w/v and 0.05 mM EDTA provided a 6.3 log₁₀ reduction compared to the control; the sixth column shows the composition used contained methylene blue at a concentration of 0.005 % w/v and 0.075 mM EDTA provided a 6.2 log₁₀ reduction compared to the control; the seventh column shows the composition used contained methylene blue at a concentration of 0.005 % w/v and 0.1 mM EDTA provided a 3.4 log₁₀ reduction compared to the control; the eight column shows the composition used contained methylene blue at a concentration of 0.005 % w/v and 0.175 mM EDTA provided a 2.3 log₁₀ reduction compared to the control.

The study also found that EDTA alone did not result in any statistically significant bacterial count reduction. None of test samples treated with EDTA alone at one of the following concentrations: 0.005 mM, 0.01 mM, 0.025 mM, 0.05 mM, 0.075 mM, 0.1 mM 0.175 mM, 0.25 mM and 0.4mM (i.e., the EDTA compositions) without light (i.e., dark incubation for 400 seconds) showed any statistically significant bacterial reduction compared to the control (i.e., test samples that received no treatment of any kind such as light, EDTA, methylene blue, or the like).

The results shown in FIG. 7 demonstrate that PDD using a composition containing a photosensitizer such as methylene blue and EDTA at a very low concentration provides enhanced photoablation against planktonic targeted microbes compared to the use of such photosensitizer alone. This planktonic study also demonstrates a dose response with concentrations of EDTA.

It should be noted that this planktonic study used a substantially lower concentration of methylene blue, a lower light energy dose rate, and a lower energy dose compared to the biofilm studies set forth in Examples I-III because the planktonic bacteria is much more sensitive to PDD treatment compared to biofilm bacteria. Using the concentration of methylene blue, light energy dose rate and/or energy dose used in the above-described biofilm studies alone (i.e., without any EDTA added to the composition) would have likely completely reduced or eradicated the planktonic MRPA and MRSA contained within each of the test samples. This would have defeated the purpose of the study which was to see whether a very low concentration of EDTA combined with a photosensitizer would provide enhanced photoablation by the photosensitizer against planktonic microbes using PDD.

The studies shown in the Examples discussed above confirmed the conventional belief that that biofilm bacteria are more resistant to PDD treatment than planktonic bacteria. As a result, a higher concentration of methylene blue with higher light energy dose and higher light energy dose rates were used in the biofilm studies compared to this planktonic study to accomplish significant photoablation.

## Claims

1. A photoablation composition comprising:
a phenothiazine; and
ethylenediaminetetraacetic acid ("EDTA") at a concentration ranging from about 0.01 mM to about 1.25 mM.

2. A composition according to claim 1 wherein the composition further includes a pharmaceutically acceptable carrier.

3. A composition according to claim 1 or claim 2 wherein the phenothiazine is
methylene blue.

4. A composition according to claim 3 wherein the methylene blue is at a concentration ranging from about 0.005% w/v to about 0.1 % w/v.

5. A composition according to claim 3 wherein the methylene blue
is at a concentration ranging from about of 0.03% w/v to about 0.05% and the EDTA is at a concentration ranging from 0.5 mM to 1.25 mM.

6. A composition according to any one of claims 1 to 4 wherein the EDTA is at a concentration ranging from 0.25 mM to about 1.25 mM.

7. A photosensitizing composition for use in the photoablation of at least one targeted organism in a photoablation method comprising:
applying a composition comprising a phenothiazine and EDTA at a concentration ranging from about 0.01 mM to about 1.25 mM to a treatment site containing the at least one targeted organism; and
applying light to the treatment site at a wavelength absorbed by the phenothiazine so as to inhibit the at least one targeted organism.

8. A photosensitising composition for use according to claim 7 wherein the composition further includes a pharmaceutically acceptable carrier.

9. A photosensitising composition for use according to claim 7 or claim 8 wherein the phenothiazine is methylene blue.

10. A photosensitising composition for use according to claim 9 wherein the methylene blue is at a concentration ranging from about 0.005% w/v to about 0.1% w/v.

11. A photosensitising composition for use according to claim 9 wherein the methylene
blue is at a concentration ranging from about of 0.03% w/v to about 0.05% and the EDTA is at a concentration ranging 0.5 mM to 1.25 mM.

12. A photosensitising composition for use according to claim 9 wherein the EDTA is at a concentration ranging from 0.25 mM to about 1.25 mM.

13. A photosensitising composition for use according to any one of claims 7 to 12 wherein the at least one targeted organism is a Gram-negative bacterial cell.

14. A photosensitising composition for use according to claim 13 wherein the Gram-negative bacterial cell is *Pseudomonas aeruginosa* or multidrug resistant *Pseudomonas aeruginosa.*

15. A photosensitising composition for use according to any one of claims 7 to 14
wherein the at least one targeted organism is in biofilm form, in planktonic form, or a tumor cell.

## Patentansprüche

1. Photoablationszusammensetzung, die umfasst:
ein Phenothiazin; und
Ethylendiamintetraacetat ("EDTA") bei einer Konzentration in einem Bereich von ungefähr 0,01 mM bis ungefähr 1,25 mM.

2. Zusammensetzung gemäß Anspruch 1, wobei die Zusammensetzung weiterhin einen pharmazeutisch annehmbaren Träger umfasst.

3. Zusammensetzung gemäß Anspruch 1 oder Anspruch 2, wobei das Phenothiazin Methylenblau ist.

4. Zusammensetzung gemäß Anspruch 3, wobei das Methylenblau bei einer Konzentration in einem Bereich von ungefähr 0,005 % w/v bis ungefähr 0,1 % w/v vorliegt.

5. Zusammensetzung gemäß Anspruch 3, wobei das Methylenblau bei einer Konzentration in einem Bereich von ungefähr 0,03 % w/v bis ungefähr 0,05 % und das EDTA bei einer Konzentration in einem Bereich von 0,5 mM bis 1,25 mM vorliegen.

6. Zusammensetzung gemäß einem der Ansprüche 1 bis 4, wobei das EDTA bei einer Konzentration in einem Bereich von 0,25 mM bis ungefähr 1,25 mM vorliegt.

7. Photosensibilisator zur Verwendung in der Photoablation mindestens eines anvisierten Organismus' in einem Photoablationsverfahren, das umfasst:
Anwendung einer Zusammensetzung, die ein Phenothiazin und EDTA bei einer Konzentration in einem Bereich von ungefähr 0,01 mM bis ungefähr 1,25 mM umfasst, auf einen Behandlungsort, der mindestens einen anvisierten Organismus umfasst; und
Anwendung von Licht auf den Behandlungsort bei einer Wellenlänge, die durch das Phenothiazin absorbiert wird, um so den mindestens einen anvisierten Organismus zu hemmen.

8. Photosensibilisator zur Verwendung gemäß Anspruch 7, wobei die Zusammensetzung weiterhin einen pharmazeutisch annehmbaren Träger umfasst.

9. Photosensibilisator zur Verwendung gemäß Anspruch 7 oder Anspruch 8, wobei das Phenothiazin Methylenblau ist.

10. Photosensibilisator zur Verwendung gemäß Anspruch 9, wobei das Methylenblau bei einer Konzentration in einem Bereich von ungefähr 0,005 % w/v bis ungefähr 0,1 % w/v vorliegt.

11. Photosensibilisator zur Verwendung gemäß Anspruch 9, wobei das Methylenblau bei einer Konzentration in einem Bereich von ungefähr 0,03 % w/v bis ungefähr 0,05 % und das EDTA bei einer Konzentration in einem Bereich von 0,5 mM bis 1,25 mM vorliegen.

12. Photosensibilisator zur Verwendung gemäß Anspruch 9, wobei das EDTA bei einer Konzentration in einem Bereich von 0,25 mM bis ungefähr 1,25 mM vorliegt.

13. Photosensibilisator zur Verwendung gemäß einem der Ansprüche 7 bis 12, wobei der mindestens eine anvisierte Organismus eine gram-negative Bakterienzelle ist.

14. Photosensibilisator zur Verwendung gemäß Anspruch 13, wobei die gram-negative Bakterienzelle *Pseudomonas aeruginosa* oder multiresistente *Pseudomonas aeruginosa* ist.

15. Photosensibilisator zur Verwendung gemäß einem der Ansprüche 7 bis 14, wobei der mindestens eine anvisierte Organismus in Form eines Biofilms, in planktonischer Form oder als eine Tumorzelle vorliegt.

## Revendications

1. Composition pour photoablation comprenant :
une phénothiazine ; et
de l'acide éthylènediaminetétraacétique (« EDTA ») à une concentration située dans la plage d'environ 0,01 mM à environ 1,25 mM.

2. Composition selon la revendication 1, laquelle composition inclut en outre un vecteur pharmaceutiquement acceptable.

3. Composition selon la revendication 1 ou la revendication 2 dans laquelle la phénothiazine est du bleu de méthylène.

4. Composition selon la revendication 3 dans laquelle le bleu de méthylène est à une concentration située dans la plage d'environ 0,005 % en p/v à environ 0,1 % en p/v.

5. Composition selon la revendication 3 dans laquelle le bleu de méthylène est à une concentration située dans la plage d'environ 0,03 % en p/v à environ 0,05% et l'EDTA est à une concentration située dans la plage de 0,5 mM à 1,25 mM.

6. Composition selon l'une quelconque des revendications 1 à 4 dans laquelle l'EDTA est à une concentration située dans la plage de 0,25 mM à environ 1,25 mM.

7. Composition photosensibilisante pour utilisation dans la photoablation d'au moins un organisme ciblé dans un procédé de photoablation comprenant :
l'application d'une composition comprenant une phénothiazine et de l'EDTA à une concentration située dans la plage d'environ 0,01 mM à environ 1,25 mM sur un site de traitement contenant l'au moins un organisme ciblé ; et
l'application de lumière sur le site de traitement à une longueur d'onde absorbée par la phénothiazine de manière à inhiber l'au moins un organisme ciblé.

8. Composition photosensibilisante pour utilisation selon la revendication 7, laquelle composition inclut en outre un vecteur pharmaceutiquement acceptable.

9. Composition photosensibilisante pour utilisation selon la revendication 7 ou la revendication 8 dans laquelle la phénothiazine est du bleu de méthylène.

10. Composition photosensibilisante pour utilisation selon la revendication 9 dans laquelle le bleu de méthylène est à une concentration située dans la plage d'environ 0,005 % en p/v à environ 0,1 % en p/v.

11. Composition photosensibilisante pour utilisation selon la revendication 9 dans laquelle le bleu de méthylène est à une concentration située dans la plage d'environ 0,03 % en p/v à environ 0,05 % et l'EDTA est à une concentration située dans la plage de 0,5 mM à 1,25 mM.

12. Composition photosensibilisante pour utilisation selon la revendication 9 dans laquelle l'EDTA est à une concentration située dans la plage de 0,25 mM à environ 1,25 mM.

13. Composition photosensibilisante pour utilisation selon l'une quelconque des revendications 7 à 12 dans laquelle l'au moins un organisme ciblé est une cellule bactérienne à Gram négatif.

14. Composition photosensibilisante pour utilisation selon la revendication 13 dans laquelle la cellule bactérienne à Gram négatif est *Pseudomonas aeruginosa* ou *Pseudomonas aeruginosa* multirésistante.

15. Composition photosensibilisante pour utilisation selon l'une quelconque des revendications 7 à 14 dans laquelle l'au moins un organisme ciblé se présente sous la forme d'un biofilm, sous une forme planctonique, ou est une cellule tumorale.
